(19) Europäisches Patentamt / European Patent Office / Office européen des brevets

(11) **EP 4 563 530 A1**

(12) **EUROPEAN PATENT APPLICATION**
published in accordance with Art. 153(4) EPC

(43) Date of publication:
**04.06.2025 Bulletin 2025/23**

(21) Application number: **23846382.2**

(22) Date of filing: **21.07.2023**

(51) International Patent Classification (IPC):
**C01F 11/18** (2006.01)　　**A61Q 1/12** (2006.01)
**A61Q 17/04** (2006.01)　　**A61Q 19/00** (2006.01)
**A61K 8/19** (2006.01)

(52) Cooperative Patent Classification (CPC):
**A61K 8/19; A61Q 1/12; A61Q 17/04; A61Q 19/00;
C01F 11/18**

(86) International application number:
**PCT/JP2023/026706**

(87) International publication number:
**WO 2024/024651 (01.02.2024 Gazette 2024/05)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.07.2022　JP 2022120529**

(71) Applicant: **Sakai Chemical Industry Co., Ltd.
Sakai-shi, Osaka 590-8502 (JP)**

(72) Inventor: **ASHIDA, Takuro
Sakai-shi, Osaka 590-0985 (JP)**

(74) Representative: **Cabinet Beau de Loménie
158, rue de l'Université
75340 Paris Cedex 07 (FR)**

(54) **SPHERICAL CALCIUM CARBONATE PARTICLES**

(57)　The present invention provides spherical calcium carbonate particles that have excellent water repellency at high temperatures and give a solidified product that is not too hard. The present invention relates to spherical calcium carbonate particles coated with a compound (a) having an acyl group, an amino group, and a -COOM group, wherein M represents a hydrogen atom, an alkali metal, an ammonium group, an organic ammonium group, or an organic amine group.

EP 4 563 530 A1

**Description**

TECHNICAL FIELD

The present invention relates to spherical calcium carbonate particles. The present invention specifically relates to spherical calcium carbonate particles useful for cosmetics and the like.

BACKGROUND ART

Cosmetics are commonly formulated with spherical particles for the purpose of achieving spreadability, high haze effect, and scrubbing effect, for example. These spherical particles can be roughly divided into those made of organic polymers such as nylon, polystyrene, and polyorganosilsesquioxanes, and those made of inorganic compounds such as silica and calcium carbonate.

In recent years, there has been a growing discussion on what is called the microplastics problem. Microplastics include spherical resin particles contained in cosmetics. The spherical resin particles discharged into the natural environment with wastewater adsorb harmful substances, which are then concentrated by plankton, fish, or the like having ingested the resin particles. The discussions have led to active development of powders and cosmetic formulations, with a focus on utilizing inorganic spherical particles that have a high specific gravity and thus are unlikely to float in the ocean and the like. Among such materials, spherical calcium carbonate is a material that has attracted attention owing to its unique soft, smooth texture and lightness.

Most inorganic compounds are hydrophilic. When blended into a cosmetic, an inorganic compound is often used as a powder coated with an organic compound. In other words, surface treating a powder with an organic compound renders the powder lipophilic, thus achieving a better texture, high dispersibility in oil, and the like. Although surface treatment with silicone was a common method in the past, cosmetic users now prefer natural products, so that surface treatment agents made from non-petroleum materials, such as fatty acids, are more popular.

Proposed surface treatment techniques for spherical calcium carbonate include the technique of treating spherical calcium carbonate with a fatty acid or silicone. For example, Patent Literature 1 discloses a method for producing surface-treated spherical calcium carbonate particles for cosmetics. The method includes adjusting an aqueous dispersion of spherical calcium carbonate particles having an average particle size falling within the range of 0.5 to 20 μm to a pH range of 9 to 11; bringing, in the aqueous dispersion of the spherical calcium carbonate particles, 2.5 parts by weight or more of a higher fatty acid salt per 100 parts by weight of the spherical calcium carbonate particles into contact with the spherical calcium carbonate particles at a temperature of 25°C or higher; and neutralizing the obtained aqueous dispersion to a pH of from 6 to 7 at room temperature. Patent Literature 2 discloses surface-treated spherical calcium carbonate particles for cosmetics, which have a hydrous silica coating on the surfaces of spherical calcium carbonate particles with a volume median diameter falling within the range of 0.5 to 20 μm, and further have a silicone oil coating thereon.

CITATION LIST

- Patent Literature

    Patent Literature 1: JP 2012-240930 A
    Patent Literature 2: JP 2014-61689 T

SUMMARY OF INVENTION

- Technical Problem

As described above, various methods have been developed as techniques for surface treating spherical calcium carbonate. However, spherical calcium carbonate surface-treated with a fatty acid as disclosed in Patent Literature 1 and the like is insufficient in water repellency in high-temperature water and has a problem with stability in a high-temperature environment. In addition, the use of a fatty acid tends to increase the adhesive force (cohesiveness) between powder particles, so that a solidified product of the powder is too hard to be used as a pressed foundation or the like. Meanwhile, the method requiring silica treatment as described in Patent Literature 2 and the like unfortunately lengthens the process and makes a cosmetic blended with silica have a rough, hard texture due to silica.

Thus, spherical calcium carbonate particles have been desired that have excellent water repellency at high temperatures and give a solidified product that is not too hard.

The present invention has been made in view of the current states above, and has an object to provide spherical calcium carbonate particles that have excellent water repellency at high temperatures and give a solidified product that is not too hard.

- Solution to Problem

The present inventors have conducted various investigations into the surface treatment of spherical calcium carbonate and have found that spherical calcium carbonate particles coated with a compound having an acyl group, an amino group, and a -COOM group (M represents a hydrogen atom, an alkali metal, an ammonium group, an organic ammonium group, or an organic amine group) have excellent water repellency at high temperatures and give a solidified product that is not too hard. The inventors have thus concluded that their findings can solve the problems above, and have thus arrived at the present invention.

The present invention includes spherical calcium carbonate particles and the like below.

(1) Spherical calcium carbonate particles coated with a compound (a) having an acyl group, an amino group, and a -COOM group, wherein M represents a hydrogen atom, an alkali metal, an ammonium group, an organic ammonium group, or an organic amine group.

(2) The spherical calcium carbonate particles according to (1) above, wherein the compound (a) is at least one of an N-acyl amino acid or a salt thereof.

(3) The spherical calcium carbonate particles according to (1) or (2) above, wherein the spherical calcium carbonate particles have a median diameter (D50) as measured by a laser diffraction/scattering particle size distribution analyzer of from 0.1 to 20 μm.

(4) The spherical calcium carbonate particles according to any one of (1) to (3) above, wherein a coating amount of the compound (a) is from 1 to 10% by weight relative to 100% by mass of the coated spherical calcium carbonate particles.

(5) A cosmetic containing the spherical calcium carbonate particles according to any one of (1) to (4) above.

- Advantageous Effects of Invention

The spherical calcium carbonate particles of the present invention having the composition above are suitable for cosmetics and the like as they have excellent water repellency at high temperatures and give a solidified product that is not too hard.

DESCRIPTION OF EMBODIMENTS

Specific preferred embodiments of the present invention are described below. The present invention is not limited to the contents below, and can be appropriately modified and applied within the scope that does not change the gist of the present invention. In addition, a combination of two or more of the individual preferred embodiments of the present invention described below also falls within the preferred embodiments of the present invention.

The spherical calcium carbonate particles of the present invention are coated with a compound (a) having an acyl group, an amino group, and a -COOM group (M represents a hydrogen atom, an alkali metal, an ammonium group, an organic ammonium group, or an organic amine group).

The compound (a) in the spherical calcium carbonate particles can be identified by observing infrared absorption by the acyl group, amino group, and -COOM group contained in the compound (a) using an infrared spectrophotometer.

The compound (a) seemingly can exhibit water repellency at high temperatures owing to hydrophobicity derived from the acyl group. The compound (a) also seemingly can remove moisture, which causes calcium carbonate aggregation, around the calcium carbonate particles owing to the amino group and the -COOM group so as to increase the fluidity between the particles, thus preventing a solidified product of the particles from becoming too hard, making it easy to take up the particles with a puff or sponge and easy to spread the particles on the skin, and giving a soft and powdery texture.

Having excellent water repellency, the spherical calcium carbonate particles of the present invention, when blended into an oil phase, can be sufficiently prevented from migrating into an aqueous phase.

The compound (a) may be any compound having an acyl group, an amino group, and a -COOM group (M represents a hydrogen atom, an alkali metal, an ammonium group, an organic ammonium group, or an organic amine group), and is preferably represented by the following formula (1):

$$R^1\text{-CONR}^2\text{-}R^3\text{-COOM} \qquad (1)$$

wherein $R^1$ represents a C1 to C30 hydrocarbon group; $R^2$ represents a hydrogen atom or a C1 to C5 hydrocarbon group; $R^3$ represents a C1 to C20 divalent hydrocarbon group which may have a functional group; and M represents a hydrogen atom, an alkali metal, an ammonium group, an organic ammonium group, or an organic amine group.

The hydrocarbon group in $R^1$ is not limited. Examples thereof include C1 to C30 aliphatic alkyl groups, C3 to C30 alicyclic alkyl groups, C2 to C30 alkenyl groups, C2 to C30 alkynyl groups, and C6 to C30 aryl groups.

Examples of the alkyl groups include aliphatic alkyl groups such as methyl, ethyl, n-propyl, n-butyl, n-pentyl (amyl), n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl, n-eicosanyl, i-propyl, sec-butyl, i-butyl, t-butyl, 1-methylbutyl, 1-ethylpropyl, 2-methylbutyl, i-amyl, neopentyl, 1,2-dimethylpropyl, 1,1-dimethylpropyl, t-amyl, 1,3-dimethylbutyl, 3,3-dimethylbutyl, 2-ethylbutyl, 2-ethyl-2-methylpropyl, 1-methylheptyl, 2-ethylhexyl, 1,5-dimethylhexyl, t-octyl, branched nonyl, decyl, undecyl, dodecyl, tridecyl, tetradecyl, pentadecyl, hexadecyl, heptadecyl, stearyl, and icosyl groups; and alicyclic alkyl groups such as cyclopropyl, cyclopropylmethyl, cyclobutyl, cyclobutylmethyl, cyclopentyl, cyclohexyl, cyclohexylmethyl, cycloheptyl, cyclooctyl, cyclohexylpropyl, cyclododecyl, norbornyl (C7), adamantyl (C10), and cyclopentylethyl groups.

Examples of the alkenyl groups include vinyl, allyl, 1-butenyl, 2-butenyl, pentenyl, hexenyl, heptenyl, octenyl, nonenyl, decenyl, dodecenyl, octadecenyl, and icosenyl groups.

Examples of the alkynyl groups include ethynyl, 1-propynyl, 2-propynyl, butynyl, pentynyl, hexynyl, heptynyl, octynyl, nonynyl, decynyl, dodecynyl, octadecynyl, and icosynyl groups.

Examples of the C6 to C30 aryl groups include phenyl; naphthyl; and aralkyl groups such as benzyl, 1-phenylethyl, 2-phenylethyl, 3-phenylpropyl, 4-phenylbutyl, styryl (Ph-CH=C-), cinnamyl (Ph-CH=CHCH2-), 1-benzocyclobutenyl, and 1,2,3,4-tetrahydronaphthyl.

The hydrocarbon group for $R^1$ has 1 to 30, preferably 3 to 28, more preferably 6 to 25, still more preferably 8 to 22, particularly preferably 10 to 18 carbon atoms.

The hydrocarbon group for $R^1$ is preferably an aliphatic alkyl group or an alkenyl group, more preferably an aliphatic alkyl group.

$R^2$ is a hydrogen atom or a C1 to C5 hydrocarbon group. The hydrocarbon group is preferably a C1 to C5 alkyl group.

$R^2$ is preferably a hydrogen atom.

$R^3$ is a C1 to C20 divalent hydrocarbon group which may have a functional group. The divalent hydrocarbon group is preferably a group obtained by removing one hydrogen atom from an alkyl group, an alkenyl group, an alkynyl group, or an aryl group, more preferably a group obtained by removing one hydrogen atom from an alkyl group. Specific examples of the alkyl group include those described above.

The hydrocarbon group for $R^3$ preferably has 1 to 15, more preferably 2 to 12, still more preferably 2 to 10, particularly preferably 2 to 8 carbon atoms.

The functional group that may be contained in $R^3$ is not limited, and may be a carboxyl group or a salt group thereof, or an amino, hydroxy, thiol, amide, guanidyl, ester, or ether group. In particular, the functional group is preferably a carboxyl group or a salt thereof, or an amino, hydroxy, thiol, or amide group, more preferably a carboxyl group or a salt thereof.

M is a hydrogen atom, an alkali metal, an ammonium group, an organic ammonium group, or an organic amine group, and is preferably a hydrogen atom or an alkali metal.

Examples of the alkali metal include lithium, sodium, and potassium, with sodium being preferred.

Examples of the organic ammonium salt forming the organic ammonium group include methylammonium salts, ethylammonium salts, dimethylammonium salts, diethylammonium salts, trimethylammonium salts, and triethylammonium salts.

Examples of the organic amine forming the organic amine group include primary amines such as methylamine, ethylamine, propylamine, n-butylamine, sec-butylamine, tert-butylamine, cyclohexylamine, benzylamine, and phenylamine; secondary amines such as dimethylamine, diethylamine, dipropylamine, dibutylamine, diisobutylamine, di-sec-butylamine, di-tert-butylamine, dicyclohexylamine, dibenzylamine, and diphenylamine; tertiary amines such as trimethylamine, triethylamine, tripropylamine, tributylamine, tricyclohexylamine, tribenzylamine, and triphenylamine; and alkanolamines such as ethanolamine, diethanolamine, and triethanolamine.

$R^3$ is preferably a group represented by the following formula (2):

$$-CH(R^4)- \qquad (2)$$

wherein $R^4$ is a hydrogen atom or a C1 to C15 hydrocarbon group which may have a functional group.

The hydrocarbon group for $R^4$ is preferably an alkyl group, an alkenyl group, an alkynyl group, or an aryl group, more preferably a C1 to C15 alkyl group. Specific examples of the alkyl group include those described above.

The hydrocarbon group for $R^3$ preferably has 1 to 12, more preferably 1 to 10, still more preferably 1 to 8, particularly preferably 1 to 6 carbon atoms.

The functional group that may be contained in $R^4$ is not limited. Specific examples and preferred embodiments thereof are similar to those of the functional group that may be contained in $R^3$.

The compound (a) is preferably at least one of an N-acylamino acid or a salt thereof.

The N-acylamino acid is a compound in which a carboxylic acid is condensed with the amino group of an amino acid.

The amino acid is at least one of twenty amino acids that form proteins. Specific examples thereof include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. Of these, neutral amino acids and acidic amino acids are preferred, acidic amino acids are more preferred, and aspartic acid and glutamic acid are particularly preferred.

The carboxylic acid is not limited. Examples thereof include saturated fatty acids such as formic acid, acetic acid, propionic acid, butyric acid, valeric acid, pivalic acid, caproic acid, enanthic acid, caprylic acid, 2-ethylhexanoic acid, pelargonic acid, capric acid, undecylic acid, lauric acid, tridecylic acid, myristic acid, pentadecylic acid, palmitic acid, margaric acid, stearic acid, nonadecylic acid, and arachidic acid; mono-unsaturated fatty acids such as acrylic acid, methacrylic acid, crotonic acid, myristoleic acid, palmitoleic acid, sapienic acid, oleic acid, elaidic acid, vaccenic acid, gadoleic acid, eicosenoic acid (paulic acid), erucic acid, and nervonic acid; di-unsaturated fatty acids such as linoleic acid, eicosadienoic acid, and docosadienoic acid; tri-unsaturated fatty acids such as $\alpha$-linolenic acid, $\gamma$-linolenic acid, pinoleic acid, eleostearic acid, mead acid, dihomo-$\gamma$-linolenic acid, and eicosatrienoic acid; tetra-unsaturated fatty acids such as stearidonic acid, arachidonic acid, eicosatetraenoic acid, and adrenic acid; penta-unsaturated fatty acids such as bosseopentaenoic acid, eicosapentaenoic acid, docosapentaenoic acid, osbond acid, clupanodonic acid, and tetracosapentaenoic acid; and hexa-unsaturated fatty acids such as docosahexaenoic acid and nisinic acid.

Of these, saturated fatty acids, mono-unsaturated fatty acids, and di-unsaturated fatty acids are preferred, and carboxylic acids in which a carboxyl group is bonded to the alkyl or alkenyl group for $R^1$ described above are more preferred.

The salt of the N-acylamino acid above is not limited. Examples thereof include alkali metal salts such as sodium salts and potassium salts, and ammonium salts. Of these, sodium salts are preferred.

Specific examples of the N-acylamino acid or a salt thereof include lauroyl glutamic acid, myristoyl glutamic acid, palmitoyl glutamic acid, stearoyl glutamic acid, caproyl glutamic acid, caprinoyl glutamic acid, oleyl glutamic acid, linoleyl glutamic acid, cocoyl glutamic acid, lauroyl aspartic acid, myristoyl aspartic acid, palmitoyl aspartic acid, stearoyl aspartic acid, caproyl aspartic acid, caprinoyl aspartic acid, oleyl aspartic acid, linoleyl aspartic acid, cocoyl aspartic acid, and salts thereof. Of these, lauroyl glutamic acid, lauroyl aspartic acid, cocoyl glutamic acid, cocoyl aspartic acid, and salts thereof are preferred.

The coating amount of the compound (a) on the spherical calcium carbonate particles of the present invention is not limited, but is preferably from 1 to 10% by mass relative to 100% by mass of the coated spherical calcium carbonate particles. This allows the effect of the present invention to be exhibited in a more sufficient manner.

The coating amount of the compound (a) is more preferably from 1 to 8% by mass, still more preferably from 2 to 7% by mass, particularly preferably from 3 to 6% by mass.

The coating amount can be calculated based on the carbon amount measurable by a carbon analyzer and the molecular weight of the compound (a).

The calcium carbonate particles of the present invention are not limited as long as they are spherical, and the sphericity, defined as the ratio of minor axis to major axis, is preferably 0.80 or higher. This improves the smoothness and blurring effect of a cosmetic containing the particles on the skin.

The spherical calcium carbonate particles of the present invention may have any particle size, and the median diameter (D50), measured with a laser diffraction/scattering particle size distribution analyzer, is preferably from 0.1 to 20 $\mu$m. This further improves the smoothness, adhesion, and texture of a cosmetic containing the particles on the skin.

The median diameter (D50) is more preferably from 0.5 to 20 $\mu$m, still more preferably from 1 to 15 $\mu$m, particularly preferably from 3 to 10 $\mu$m.

The spherical calcium carbonate particles of the present invention preferably contain 90% or more vaterite crystals, more preferably 98 to 100% vaterite crystals.

The proportion of vaterite crystals in calcium carbonate can be calculated, as already well known, in accordance with the following formula (3) (M. S. Rao, Bull. Chem. Soc. Japan, 46, 1414 (1973)).

$$\text{Proportion F(v) of vaterite crystals} = f(v) \times 100 \quad (3)$$

Here, $f(v) = 1 - I_{104(c)}/(I_{110(v)} + I_{112(v)} + I_{114(v)} + I_{104(c)})$, wherein $I_{104(c)}$ is the X-ray diffraction intensity of 104 plane of calcite, $I_{110(v)}$ is the X-ray diffraction intensity of 110 plane of vaterite, $I_{112(v)}$ is the X-ray diffraction intensity of 112 plane of vaterite, and $I_{114(v)}$ is the X-ray diffraction intensity of 114 plane of vaterite.

The spherical calcium carbonate particles of the present invention give a solidified product that is not too hard, and preferably have a penetration load measured by the following method of from 1 to 15 mN, more preferably from 1 to 10 mN.

(Method for measuring penetration load)

Spherical calcium carbonate particles are placed in a container up to the rim of the container, and a load of 6 MPa is applied to the particles using a hand press for 20 seconds. After the load is released, a load of 6 MPa is again applied for 20 seconds to produce a molded product, and the load when a needle with a diameter of 1 mm is inserted 1 mm into the molded product is measured three times using a rheometer. The average value of the three measurements is calculated.

The spherical calcium carbonate particles of the present invention have excellent water repellency at high temperatures, and preferably include 90% or more spherical calcium carbonate particles that float on the water surface in the following water repellency evaluation test at 80°C. This allows the emulsion system of a cosmetic containing the particles to remain stable even when the cosmetic is exposed to high temperatures.

(Evaluation of water repellency at 80°C)

Spherical calcium carbonate particles in an amount of 1 g are gently placed on the surface of 100 ml of pure water at 80°C, followed by stirring at 60 rpm with a magnetic stirrer for 1 minute. The water repellency is then evaluated.

The spherical calcium carbonate particles of the present invention may be produced by any method. The method preferably includes a step of mixing calcium carbonate as a raw material with the compound (a) above (hereinafter also referred to as a mixing step).

The calcium carbonate used as a raw material in the mixing step is not limited as long as spherical calcium carbonate particles are obtained, and the sphericity of the particles is preferably 0.80 or higher.

The calcium carbonate as a raw material may have any particle size, and the median size (D50) measured by the method above is preferably from 0.1 to 20 $\mu$m.

With the particle shape and particle size as described above, powder particles with a pleasant texture can be obtained.

The mixing step is not limited as long as calcium carbonate as a raw material and the compound (a) above are mixed, and preferably includes adding calcium carbonate as a raw material and the compound (a) above to solvent(s), followed by mixing. In a more preferred embodiment, at least one of calcium carbonate or the compound (a) is dispersed or dissolved in solvent(s), followed by mixing. In a still more preferred embodiment, the compound (a) or a solution of the compound (a) is added to a dispersion of calcium carbonate, followed by mixing.

The solvent in which at least one of calcium carbonate or the compound (a) is dispersed or dissolved is not limited. Examples thereof include water and watersoluble organic solvents, including alcohols such as methanol, ethanol, n-propanol, and i-propanol, polyhydric alcohols such as ethylene glycol, diethylene glycol, and glycerin, pyrrolidone-based solvents such as N-methylpyrrolidone, and ketone-based solvents such as acetone. The preferred solvent is water.

In the method for producing the spherical calcium carbonate particles, an embodiment in which the compound (a) or an aqueous solution of the compound (a) is added to an aqueous dispersion of calcium carbonate, followed by mixing, is a preferred embodiment.

The amount of the compound (a) used in the mixing step is not limited and can be adjusted so as to achieve a preferred coating amount. The amount is preferably from 1 to 10% by mass, more preferably from 2 to 8% by mass, relative to 100% by mass of the calcium carbonate as a raw material.

In the mixing step, when the calcium carbonate as a raw material is in the form of a dispersion, the proportion of the calcium carbonate in the dispersion is preferably from 100 to 350 g, more preferably from 150 to 300 g, in 1 L of the solvent.

The temperature in the mixing step is not limited, and is preferably from 10°C to 100°C, more preferably from 20°C to 80°C, still more preferably from 25°C to 40°C. This can suppress the change in shape of the calcium carbonate particles from spherical to cubic and the costs in a more sufficient manner.

In the method for producing the spherical calcium carbonate particles of the present invention, the mixing step is preferably followed by stirring.

The preferred temperature range in the stirring step is similar to the preferred temperature range in the mixing step.

The stirring duration in the stirring step is not limited, and is preferably from 1 minute to 24 hours, more preferably from 30 minutes to 18 hours, still more preferably from 1 hour to 10 hours.

In the method for producing the spherical calcium carbonate particles of the present invention, the mixing step (and the stirring step) is preferably followed by a washing step and a drying step.

The washing step is not limited, and preferably includes filtering the spherical calcium carbonate particles obtained in the mixing step (and the stirring step) and washing the particles with water. More preferably, the obtained spherical calcium carbonate particles are repeatedly filtered and washed with water until the electrical conductivity of the filtrate is 100 $\mu$S/cm or less. This can more sufficiently suppress the occurrence of foaming due to the remaining compound (a) or the like in formulation of the spherical calcium carbonate particles.

The drying temperature in the drying step is preferably from 50°C to 150°C, more preferably from 70°C to 120°C, still more preferably from 80°C to 110°C.

The drying duration in the drying step is preferably from 1 to 72 hours, more preferably from 10 to 60 hours, still more preferably from 24 to 48 hours.

The spherical calcium carbonate particles of the present invention have excellent water repellency at high temperatures, give a solidified product that is not too hard, are easy to spread on the skin, and provide a soft and powdery texture, thus being suitable for use in cosmetics and the like.

A cosmetic containing the spherical calcium carbonate particles of the present invention is also one aspect of the present invention.

Examples of the cosmetic of the present invention include foundations, makeup bases, emulsions, creams, eyeshadows, blushes, mascaras, lipsticks, and sunscreens. The cosmetic of the present invention may be in any form, such as an oil-based cosmetic, a water-based cosmetic, an O/W type cosmetic, or a W/O type cosmetic. The cosmetic can be particularly suitable as a makeup product such as a foundation, a makeup base, or an eyeshadow, a skincare product such as a toner, a cream, an emulsion, or a beauty serum, or a sunscreen agent.

The cosmetic of the present invention may contain any water-based component and/or oil-based component that can be used in the cosmetic field as well as the spherical calcium carbonate particles. The water-based component and oil-based component are not limited, and may include, for example, oils, surfactants, moisturizers, higher alcohols, sequestering agents, natural and synthetic polymers, watersoluble and oil-soluble polymers, ultraviolet screening agents, various extracts, inorganic and organic pigments, inorganic and organic clay mineral powders and other various powders, inorganic and organic pigments treated with metal soaps or silicone, organic dyes and other colorants, preservatives, antioxidants, pigments, thickeners, pH adjusters, fragrances, cooling agents, antiperspirants, disinfectants, skin activators, and other ingredients. Specifically, for example, the desired cosmetic can be produced by a conventional method by optionally blending one or two or more of the ingredients listed in JP 2014-080392 A. The amounts of these ingredients are not limited as long as they do not impair the effect of the present invention.

EXAMPLES

The present invention will be described in more detail below with reference to examples. The present invention is not limited to these examples. Unless otherwise specified, "%" and "wt%" mean "% by weight (% by mass)". The physical properties are measured as follows.

(Median diameter D50)

The volume-based median diameter D50 was measured using the laser diffraction/scattering particle size distribution analyzer LA-750 available from Horiba, Ltd.

(Electrical conductivity of filtrate)

The electrical conductivity was measured using the conductivity meter ES-12 available from Horiba Ltd.

(Sphericity of spherical calcium carbonate particles)

One hundred particles were randomly selected on an SEM photograph and the major axis and minor axis of each particle were measured to determine the minor axis/major axis ratio.

(Evaluation of water repellency at 25°C)

Each powder in an amount of 1 g was gently placed on the surface of 100 mL of pure water at room temperature, 25°C, followed by stirring at 60 rpm for 1 minute using a magnetic stirrer. The water repellency was then visually evaluated based on the following criteria.

∘: Almost the entire amount of the powder is floating on the water surface.
△: A half or more of the powder is floating on the water surface, but part of it is merged with water.
✕: Most of the powder is merged with water.

(Evaluation of water repellency at 80°C)

Each powder in an amount of 1 g was gently placed on the surface of 100 ml of pure water at 80°C, followed by stirring at 60 rpm for 1 minute using a magnetic stirrer. The water repellency was then visually evaluated based on the following criteria.

○: Almost the entire amount of the powder is floating on the water surface.
△: A half or more of the powder is floating on the water surface, but part of it is merged with water.
×: Most of the powder is merged with water.

(Coating amount of compound (a))

The amount of carbon detected using the carbon analyzer EMIA-110 (available from Horiba, Ltd.) at a temperature setting of 500°C was divided by the weight ratio of the total carbon in a molecule to the molecular weight of each surface treatment agent (compound (a)) (the weight ratio of the total carbon to the dimethylsiloxy unit in Comparative Example 3). The calculated value was taken as the coating amount of the compound (a).

(Evaluation of penetration load)

Powder was placed in a rectangular aluminum dish up to the rim of the dish, and a load of 6 MPa was applied to the powder for 20 seconds using a hand press. After the load was released, a load of 6 MPa was again applied for 20 seconds to produce a molded product. Using a rheometer (CR-100 available from Sun Scientific Co., Ltd.), the load when a needle with a diameter of 1 mm was inserted 1 mm into the molded product was measured three times, and the average value of the three measurements was calculated.

(Evaluation of viscosity when thickener is used in combination)

Powder in an amount of 2 g was dispersed in 5 g of 1,3-butylene glycol. The resulting dispersion was added to 10 g of a thickener diluted with 90 g of water, followed by thorough dispersion with a stirrer. The thickener was a 5 wt% aqueous solution of an (ammonium acryloyldimethyltaurate/vinyl pyrrolidone) copolymer (Aristoflex AVC, available from Clariant). The viscosity of the resulting dispersion after standing for 24 hours was measured using a B type viscometer. The viscosity of the thickener alone is 11200 mPa·s.

<Example 1>

Spherical calcium carbonate (particle size 5 $\mu$m, Calmaru SCS-M5 available from Sakai Chemical Industry Co., Ltd.) in an amount of 100 g was dispersed in 500 ml of water. The resulting dispersion was stirred at 25°C for 30 minutes, and then 20 g of a 25 wt% aqueous solution of sodium lauroyl aspartate (AminoFoamer FLDS-L, available from Asahi Kasei Finechem Co., Ltd.) was added. The resulting mixture was stirred for 1 hour, and then filtered and washed with water (final electrical conductivity 92 $\mu$S/cm). The obtained wet cake was dried overnight at 105°C in a dryer to obtain Powder 1.

<Example 2>

Spherical calcium carbonate (particle size 5 $\mu$m, Calmaru SCS-M5 available from Sakai Chemical Industry Co., Ltd.) in an amount of 100 g was dispersed in 500 ml of water. The resulting dispersion was stirred at 25°C for 30 minutes, and then 4 g of sodium cocoyl glutamate (AMISOFT CS-11, available from Ajinomoto Healthy Supply Co., Inc.) was added. The resulting mixture was stirred for 1 hour, and then filtered and washed with water (final electrical conductivity 95 $\mu$S/cm). The obtained wet cake was dried overnight at 105°C in a dryer to obtain Powder 2.

<Example 3>

Powder 3 was obtained in a manner similar to that in Example 1, except that the amount of the 25 wt% aqueous solution of sodium lauroyl aspartate was changed to 32 g. The final electrical conductivity after washing with water was 70 $\mu$S/cm.

<Example 4>

Powder 4 was obtained in a manner similar to that in Example 2, except that the amount of sodium cocoyl glutamate was changed to 2 g. The final electrical conductivity after washing with water was 86 μS/cm.

<Example 5>

Powder 5 was obtained in a manner similar to that in Example 1, except that the amount of the 25 wt% aqueous solution of sodium lauroyl aspartate was changed to 2 g. The final electrical conductivity after washing with water was 90 μS/cm.

<Comparative Example 1>

Spherical calcium carbonate (particle size 5 μm, Calmaru SCS-M5 available from Sakai Chemical Industry Co., Ltd.) in an amount of 100 g was dispersed in 500 ml of water. In a separate container, 5 g of sodium stearate (available from Fujifilm Wako Pure Chemical Corporation) was dissolved in 858 ml of warm water. Next, this solution was added to the calcium carbonate dispersion at a rate of 50 ml/min, and then the mixture was heated to 70°C and stirred for 1 hour. Thereafter, the mixture was cooled to 25°C, neutralized to pH 7 with dilute hydrochloric acid, and stirred for 30 minutes. The mixture was then filtered and washed with water (final electrical conductivity 93 μS/cm). The obtained wet cake was dried overnight at 105°C in a dryer to obtain Powder 6.

<Comparative Example 2>

Spherical calcium carbonate (particle size 5 μm, Calmaru SCS-M5 available from Sakai Chemical Industry Co., Ltd.) in an amount of 100 g was dispersed in 500 ml of water. In a separate container, 5 g of potassium myristate (available from Fujifilm Wako Pure Chemical Corporation) was dissolved in 858 ml of warm water. Next, this solution was added to the calcium carbonate dispersion at a rate of 50 ml/min, and then the mixture was heated to 70°C and stirred for 1 hour. Thereafter, the mixture was cooled to 25°C, neutralized to pH 7 with dilute hydrochloric acid, and stirred for 30 minutes. The mixture was then filtered and washed with water (final electrical conductivity 75 μS/cm). The obtained wet cake was dried overnight at 105°C in a dryer to obtain Powder 7.

<Comparative Example 3>

Spherical calcium carbonate (particle size 5 μm, Calmaru SCS-M5 available from Sakai Chemical Industry Co., Ltd.) in an amount of 100 g was mixed with a solution of 5 g of hydrogen dimethicone in 50 ml of isopropyl alcohol, and the resulting mixture was thoroughly kneaded for 1 hour. The kneaded product was dried overnight at 105°C in a dryer to obtain Powder 8.

<Comparative Example 4>

Spherical calcium carbonate (particle size 5 μm, Calmaru SCS-M5 available from Sakai Chemical Industry Co., Ltd.) was used as Powder 9.

The evaluation results of various physical properties in Examples 1 to 5 and Comparative Examples 1 to 4 are shown in Table 1.

[Table 1]

| | Example 1 | Example 2 | Example 3 | Example 4 | Example 5 | Comparative Example 1 | Comparative Example 2 | Comparative Example 3 | Comparative Example 4 |
|---|---|---|---|---|---|---|---|---|---|
| | Powder 1 | Powder 2 | Powder 3 | Powder 4 | Powder 5 | Powder 6 | Powder 7 | Powder 8 | Powder 9 |
| Sphericity | 0.92 | 0.91 | 0.92 | 0.95 | 0.93 | 0.94 | 0.93 | 0.92 | 0.92 |
| Water repellency at 25°C | O | O | O | O | Δ | O | O | × | × |
| Water repellency at 80°C | O | O | O | O | Δ | × | × | - | - |
| Coating amount (%) of compound (a) | 3.5 | 3.6 | 6.4 | 1.5 | 0.3 | 4.4 | 3.2 | 4.2 | - |
| Penetration load (mN) | 2 | 5 | 2 | 6 | 8 | 41 | 20 | - | 9 |
| Viscosity (mPa·s) when thickener is used in combination | 12,300 | 10,500 | 11,200 | 8,400 | 1,020 | 4,200 | - | - | 150 |

The evaluation results in Table 1 show that in Examples 1 to 5, the spherical calcium carbonate particles coated with the compound (a) have excellent water repellency even at high temperatures and have a small penetration load, and thus achieve the effect of giving a product that is not too hard.

The results also revealed that the spherical calcium carbonate particles of Examples 1 to 4, when used in combination with a thickener, did not interfere with the thickener and maintained a viscosity equivalent to that of the thickener alone (11200 mPa·s). This shows that the surface treatment in the present invention is useful also when the particles are used in combination with a thickener in an O/W emulsion or the like, thus enabling the spherical calcium carbonate particles to be stably blended.

<Formulation Example 1>

An O/W emulsion foundation having the composition shown in Table 2 below was prepared.

[Table 2]

| (Ingredient) | (wt%) |
|---|---|
| 1. Stearic acid | 0.7 |
| 2. Behenyl alcohol | 0.5 |
| 3. Glyceryl stearate | 0.5 |
| 4. Squalane | 8.0 |
| 5. Caprylic/capric triglyceride | 4.0 |
| 6. Isotridecyl isononanoate | 2.0 |
| 7. Sorbitan sesquioleate | 0.5 |
| 8. (Acrylates/alkyl acrylate (C10-30)) crosspolymer (2% gel) | 5.0 |
| 9. Polysorbate 80 | 1.2 |
| 10. (Ammonium dimethyl taurate acrylate/vinylpyrrolidone) copolymer (5% gel) | 10.0 |
| 11. Xanthan gum (2% gel) | 2.0 |
| 12. 1,3-Butylene glycol | 7.0 |
| 13. Triethanolamine | 0.75 |
| 14. Water | 20.65 |
| 15. Powder 1 | 5.0 |
| 16. Titanium oxide dispersion (Note 1) | 11.2 |
| 17. Iron oxide (yellow) dispersion (Note 2) | 2.2 |
| 18. Iron oxide (red) dispersion (Note 3) | 0.6 |
| 19. Iron oxide (black) dispersion (Note 4) | 0.2 |
| 20. Water | 18.0 |

(Note 1) DIP-T1(N) (available from Sakai Chemical Industry Co., Ltd.): titanium oxide 75 wt%, hydrogen dimethicone 1 wt%, 1,3-butylene glycol 22 wt%, PEG-9 dimethicone 3 wt%
(Note 2) DIP-Y1(N) (available from Sakai Chemical Industry Co., Ltd.): yellow iron oxide 59 wt%, hydrogen dimethicone 1 wt%, 1,3-butylene glycol 37.5 wt%, PEG-9 dimethicone 2.5 wt%
(Note 3) DIP-R1(N) (available from Sakai Chemical Industry Co., Ltd.): red iron oxide 49 wt%, hydrogen dimethicone 1 wt%, 1,3-butylene glycol 48.5 wt%, PEG-9 dimethicone 1.5 wt%
(Note 4) DIP-K1(N) (available from Sakai Chemical Industry Co., Ltd.): black iron oxide 59 wt%, hydrogen dimethicone 1 wt%, 1,3-butylene glycol 37.5 wt%, PEG-9 dimethicone 2.5 wt%

(Production method)

A: Ingredients 1 to 7 were mixed homogeneously and heated to 80°C.
B: Ingredients 8 to 14 were mixed homogeneously and heated to 80°C.
C: A was added to B and emulsified.
D: Ingredients 16 to 20 were mixed homogeneously.
E: Ingredient 15 and D were added to C at 60°C or lower and mixed homogeneously.

With Powder 1 blended, an O/W emulsion foundation having excellent settling resistance and an excellent texture was successfully obtained.

<Formulation Example 2>

A W/O cream having the composition shown in Table 3 below was prepared.

[Table 3]

| (Ingredient) | (wt%) |
| --- | --- |
| 1. Dimethylpolysiloxane | 5.0 |
| 2. Methylphenylpolysiloxane | 5.0 |
| 3. Squalane | 8.0 |
| 4. Neopentyl glycol dioctanoate | 3.0 |
| 5. PEG-10 dimethicone | 3.0 |
| 6. Powder2 | 5.0 |
| 7. Glycerin | 10.0 |
| 8. Water | 61.0 |

(Production method)

A: Ingredients 1 to 5 were mixed and homogenized, and then Ingredient 6 was added and mixed homogenously.
B: Ingredient 7 and Ingredient 8 were mixed and stirred.
C: B was gradually added to A with stirring.

With Powder 2 blended, a W/O cream having excellent settling resistance and an excellent texture was obtained.

<Formulation Example 3>

A W/O sunscreen emulsion having the composition shown in Table 4 below was prepared.

[Table 4]

| (Ingredient) | (wt%) |
| --- | --- |
| 1. Cyclopentasiloxane | 15.0 |
| 2. Olefin oligomer | 10.0 |
| 3. Dimethylpolysiloxane | 2.0 |
| 4. PEG-9 dimethicone | 1.0 |
| 5. Neopentyl glycol dioctanoate | 5.0 |
| 6. Diisopropyl sebacate | 5.0 |

(continued)

| (Ingredient) | (wt%) |
|---|---|
| 7. 4-Tert-butyl-4'-methoxydibenzoylmethane | 2.5 |
| 8. Trimethylstearyl ammonium bentonite | 0.2 |
| 9. Powder 1 | 5.0 |
| 10. Silicone-treated zinc oxide (Note 5) | 15.0 |
| 11. Water | 35.3 |
| 12. Glycerin | 3.0 |
| 13. Hydroxyethyl cellulose | 0.5 |
| 14. Phenoxyethanol | 0.5 |

(Note 5) FINEX-52W-LP2 (available from Sakai Chemical Industry Co., Ltd.): zinc oxide 86 wt%, silica 10 wt%, hydrogen dimethicone 4 wt%

(Production method)

A: Ingredients 1 to 5 were mixed homogenously.
B: Ingredients 6 and 7 were heated to 80°C, mixed and dissolved, and added to A.
C: Ingredients 8 to 10 were added to B and mixed vigorously to be thoroughly dispersed.
D: Ingredients 11 to 14 were heated to 95°C, mixed and dissolved, and gradually mixed into C with stirring.
E: After being mixed thoroughly and homogeneously, the mixture was cooled to 25°C, and thus a sunscreen emulsion was obtained.

With Powder 1 used, a W/O sunscreen emulsion having excellent settling resistance and an excellent texture was obtained.

<Formulation Example 4>

A powder foundation having the composition shown in Table 5 below was prepared.

[Table 5]

| (Ingredient) | (wt%) |
|---|---|
| 1. Powder 1 | 10.5 |
| 2. Metal soap-treated flake-shaped barium sulfate (Note 6) | 30.0 |
| 3. Silicone-treated zinc oxide (Note 7) | 2.5 |
| 4. Silicone-treated titanium oxide (Note 8) | 5.5 |
| 5. Silicone-treated mica (Note 9) | 13.0 |
| 6. Silicone-treated synthetic golden mica (Note 10) | 12.5 |
| 7. Silicone-treated talc (Note 11) | 8.1 |
| 8. Lauroyl lysine | 5.0 |
| 9. Silicone-treated red iron oxide (Note 12) | 0.4 |
| 10. Silicone-treated yellow iron oxide (Note 13) | 1.8 |
| 11. Silicone-treated black iron oxide (Note 14) | 0.2 |
| 12. Squalane | 2.5 |
| 13. (Dimethicone/phenylvinyldimethicone) crosspolymer, diphenylsiloxyphenyl trimethicone | 6.0 |

(continued)

| (Ingredient) | (wt%) |
|---|---|
| 14. (Acrylates/stearyl acrylate/dimethicone methacrylate) copolymer | 2.0 |

(Note 6) HG-LFP (available from Sakai Chemical Industry Co., Ltd.): barium sulfate 98.9 wt%, palmitic acid 1.0 wt%, magnesium hydroxide 0.1 wt%

(Note 7) FINEX-50S-LP2 (available from Sakai Chemical Industry Co., Ltd.): zinc oxide 96 wt%, hydrogen dimethicone 4 wt%

(Note 8) MKR-1S (available from Sakai Chemical Industry Co., Ltd.): titanium oxide 98.5 wt%, hydrogen dimethicone 1.5 wt%

(Note 9) Y-2300X (available from Yamaguchi Mica Co., Ltd.): Mica 98 wt%, hydrogen dimethicone 2 wt%

(Note 10) PDM-5L(S) (available from Topy Industries, Ltd.): golden mica 98 wt%, hydrogen dimethicone 2 wt%

(Note 11) SA-talc JA-46R (available from Miyoshi Kasei, Inc.): talc 98 wt%, hydrogen dimethicone 2 wt%

(Note 12) SI-red R-516PS LHC (available from Miyoshi Kasei, Inc.): red iron oxide 98 wt%, hydrogen dimethicone 2 wt%

(Note 13) SI-yellow LL-100P LHC (available from Miyoshi Kasei, Inc.): yellow iron oxide 98 wt%, hydrogen dimethicone 2 wt%

(Note 14) SI-black BL-100P LHC (available from Miyoshi Kasei, Inc.): black iron oxide 98 wt%, hydrogen dimethicone 2 wt%

(Production method)

A: Ingredients 1 to 14 were mixed in a mixer.
B: The resulting mixture was pressed using a press.

With Powder 1 used, a powder foundation having a good texture with no roughness was successfully obtained.

<Formulation Example 5>

A powder foundation having the composition shown in Table 6 below was prepared.

[Table 6]

| (Ingredient) | (wt%) |
|---|---|
| 1. Powder2 | 10.0 |
| 2. Silicone-treated mica (see Note 9 above) | 30.8 |
| 3. Silicone-treated talc (see Note 11 above) | 15.0 |
| 4. Silicone-treated titanium oxide (see Note 8 above) | 10.0 |
| 5. Silicone-treated synthetic golden mica (see Note 10 above) | 10.0 |
| 6. Silicone-treated red iron oxide (see Note 12 above) | 0.5 |
| 7. Silicone-treated yellow iron oxide (see Note 13 above) | 2.0 |
| 8. Silicone-treated black iron oxide (see Note 14 above) | 0.2 |
| 9. Polymethylmethacrylate | 10.0 |
| 10. Dimethicone (500 cst) | 5.0 |
| 11. Isononyl isononanoate | 3.0 |
| 12. Squalane | 2.0 |
| 13. Vaseline | 1.0 |

(continued)

| (Ingredient) | (wt%) |
|---|---|
| 14. Phenoxyethanol | 0.5 |

(Production method)

A: Ingredients 1 to 14 were mixed in a mixer.
B: Light liquid isoparaffin in an amount of 50 parts was added to 100 parts by weight of A and mixed homogeneously in the mixer.
C: The slurry of B was loaded into a dish and compression molded with suctioning in vacuum.
D: The molded product of C was dried at 70°C for 10 hours to obtain a powder foundation.

With Powder 2 used, a powder foundation having a good texture was successfully obtained.

**Claims**

1. Spherical calcium carbonate particles coated with a compound (a) having an acyl group, an amino group, and a -COOM group, wherein M represents a hydrogen atom, an alkali metal, an ammonium group, an organic ammonium group, or an organic amine group.

2. The spherical calcium carbonate particles according to claim 1,
   wherein the compound (a) is at least one of an N-acyl amino acid or a salt thereof.

3. The spherical calcium carbonate particles according to claim 1 or 2,
   wherein the spherical calcium carbonate particles have a median diameter (D50) as measured by a laser diffraction/scattering particle size distribution analyzer of from 0.1 to 20 $\mu$m.

4. The spherical calcium carbonate particles according to claim 1 or 2,
   wherein a coating amount of the compound (a) is from 1 to 10% by mass relative to 100% by mass of the coated spherical calcium carbonate particles.

5. A cosmetic comprising the spherical calcium carbonate particles according to claim 1 or 2.

## INTERNATIONAL SEARCH REPORT

| International application No. |
| --- |
| **PCT/JP2023/026706** |

| A. | CLASSIFICATION OF SUBJECT MATTER |
| --- | --- |

*C01F 11/18*(2006.01)i; *A61Q 1/12*(2006.01)i; *A61Q 17/04*(2006.01)i; *A61Q 19/00*(2006.01)i; *A61K 8/19*(2006.01)i
FI:   C01F11/18 J; A61K8/19; A61Q1/12; A61Q19/00; A61Q17/04

According to International Patent Classification (IPC) or to both national classification and IPC

| B. | FIELDS SEARCHED |
| --- | --- |

Minimum documentation searched (classification system followed by classification symbols)

C01F11/18; A61Q1/12; A61Q17/04; A61Q19/00; A61K8/19

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| Y | WO 2014/061689 A1 (SAKAI CHEMICAL INDUSTRY CO., LTD.) 24 April 2014 (2014-04-24)<br>paragraphs [0007], [0008], [0011], [0027] | 1-5 |
| Y | WO 2010/113899 A1 (FUJIFILM CORP.) 07 October 2010 (2010-10-07)<br>paragraphs [0037], [0038] | 1-5 |
| A | JP 2016-164249 A (KEY TRANDING CO., LTD.) 08 September 2016 (2016-09-08)<br>paragraphs [0071], [0075], [0081], [0092], [0093] | 1-5 |
| A | WO 2021/200352 A1 (KAO CORP.) 07 October 2021 (2021-10-07)<br>paragraphs [0061]-[0064] | 1-5 |
| A | JP 2022-063984 A (ARUBION KK) 25 April 2022 (2022-04-25)<br>paragraphs [0053]-[0055], [0062], [0063], [0069], [0070] | 1-5 |

☐ Further documents are listed in the continuation of Box C.      ☑ See patent family annex.

| * | Special categories of cited documents: | | |
| --- | --- | --- | --- |
| "A" | document defining the general state of the art which is not considered to be of particular relevance | "T" | later document published after the international filing date or priority date and not in conflict with the application but cited to understand the principle or theory underlying the invention |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **18 August 2023** | **05 September 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/026706**

| Patent document cited in search report | Publication date (day/month/year) | Patent family member(s) | Publication date (day/month/year) |
|---|---|---|---|
| WO 2014/061689 A1 | 24 April 2014 | US 2015/0290094 A1 paragraphs [0009]-[0011], [0018], [0038] EP 2910237 A1 KR 10-2015-0067232 A CN 104822359 A | |
| WO 2010/113899 A1 | 07 October 2010 | US 2012/0027830 A1 paragraphs [0061], [0062] EP 2415447 A1 CN 102361625 A KR 10-2011-0133493 A | |
| JP 2016-164249 A | 08 September 2016 | (Family: none) | |
| WO 2021/200352 A1 | 07 October 2021 | JP 2021-165264 A EP 4129410 A1 paragraphs [0049]-[0056] KR 10-2022-0156583 A CN 115427010 A TW 202203896 A | |
| JP 2022-063984 A | 25 April 2022 | (Family: none) | |

Form PCT/ISA/210 (patent family annex) (January 2015)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012240930 A
- JP 2014061689 T

- JP 2014080392 A

**Non-patent literature cited in the description**

- **M. S. RAO**. *Bull. Chem. Soc. Japan*, 1973, vol. 46, 1414